# FASCICULE DE BREVET EUROPEEN

(11) **EP 1 991 170 B1**
(45) Date de publication et mention de la délivrance du brevet: **12.10.2016**
(21) Numéro de dépôt: 07731067.0
(22) Date de dépôt: 01.03.2007
(51) Int. Cl.: A61F 2/30, A61L 24/00

(54) **IMPLANT PROVISOIRE COMPRENANT DEUX ACTIFS**
TEMPORÄRES IMPLANTAT MIT ZWEI WIRKSTOFFEN
TEMPORARY IMPLANT COMPRISING TWO ACTIVE SUBSTANCES

(30) Priorité: 03.03.2006 FR 0601899
(43) Date de publication de la demande: 19.11.2008
(73) Titulaire: Teknimed SAS, 65500 Vic en Bigorre (FR)
(72) Inventeur: LEONARD, Alain, 65500 Caixon (FR); LAVERNE, Claudine, 65500 Caixon (FR); OGLAZA, Jean-François, F-31500 Toulouse (FR)
(74) Mandataire: Cabinet Morelle & Bardou SC
(86) Numéro de dépôt international: PCT/FR2007/000367
(87) Numéro de publication internationale: WO 2007/099232

(56) Documents cités:
- EP-A- 1 166 724
- EP-A- 1 457 172
- WO-A-01/76512
- DE-U1- 29 703 971
- US-A- 5 681 289
- US-B1- 6 245 111
- C. SCHOELLER, S. FUERDERER, J.-D. ROMPE, A. ECKARDT: "individual bone cement spacers for septic hip revision" ARCH ORTHOP TRAUMA SURG, vol. 123, 1 mai 2003 (2003-05-01), pages 254-259, XP002408999
- E. BERTAZZONI MINELLI ET COLL.: "Release of gentamicin and vancomycin from temporary human hip spacers in two-stage revision of infected arthroplasty" JOURNAL OF ANTIMICROBIAL CHEMOTHERAPY, vol. 53, 2004, pages 329-334, XP002409000 cité dans la demande

## Description

La présente invention appartient au domaine des implants articulaires provisoires destinés à maintenir un espace convenable pour la durée nécessaire aux opérations de remplacement d'une prothèse permanente. Ces implants provisoires sont aussi appelés "espaceurs".

L'invention a pour objet un dispositif espaceur prêt à l'emploi, formé de deux modules assemblés qui apportent au moins deux substances actives différentes, telles que deux antibiotiques.

Chez les patients équipés d'une articulation artificielle, comme par exemple une prothèse de hanche, il n'est pas rare de devoir procéder au remplacement de l'élément implanté, soit du fait de sa détérioration mécanique, soit du fait de complications infectieuses atteignant la zone d'implantation et entraînant un descellement de la prothèse. Il est alors nécessaire de retirer la prothèse et de réaliser un traitement antiseptique et antibiotique avant de remettre en place une implant permanent. L'ensemble de cette opération appelée "reprise de prothèse en deux temps", dure plusieurs semaines, voire plusieurs mois, car il est indispensable avant toute réimplantation, de traiter complètement l'infection par une antibiothérapie par voix générale associée à une antibiothérapie locale.

Pour éviter que les tissus (muscles, tendons, os) ne viennent occuper l'espace libéré par le retrait de la prothèse permanente, on place un espaceur remplaçant provisoirement la prothèse dans la zone articulaire. Ainsi, on réduit le risque de formation d'un hématome et de surinfection, et le membre est mieux stabilisé. En outre le patient garde une certaine mobilité durant la période transitoire et, ultérieurement, la mise en placé de la prothèse définitive est facilitée.

Les espaceurs sont communément réalisés dans des matériaux relativement bon marché, tels que les ciments polymères, le plus souvent le polyméthylméthacrylate (en abrégé PMMA). L'emploi d'un tel polymère présente l'avantage de permettre à la fois le maintien de la zone d'implantation et le traitement local de l'infection. En effet, du fait de sa structure réticulée, il peut être combiné à un agent antibiotique qui diffuse dans les tissus environnants et assure ainsi un traitement local prolongé de l'infection. L'antibiotique est libéré progressivement par diffusion dans les fluides corporels qui le transportent ensuite vers les tissus voisins.

L'antibiotique systématiquement employé en chirurgie osseuse est la gentamicine, qui est un antibiotique à spectre large. Cependant face à l'accroissement du taux de résistance des bactéries isolées dans les cas d'infection de hanche notamment, des études récentes ont envisagé l'emploi d'une combinaison d'antibiotiques dans les espaceurs en PMMA, comme par exemple de gentamicine et de vancomycine (Bertazzoni Minelli E. et coll., Journal of Antimicrobial Chemotherapy, 2004, 53, pp. 329-334). Pour cela, des espaceurs prémoulés chargés en gentamicine du commerce ont été percés de trous et ceux-ci ont été remplis d'un ciment chargé en vancomycine, cette technique permettant d'éviter les interférences dans la libération des deux antibiotiques par le PMMA et d'ajuster la vitesse d'élution pour chaque antibiotique. Ces essais ont démontré l'efficacité de l'emploi combiné de deux antibiotiques dans le traitement local des infections au niveau des prothèses articulaires lors d'une reprise en deux temps, y compris vis-à-vis de souches résistantes aux traitements conventionnels. Cependant l'introduction du second antibiotique dans un espaceur préchargé en gentamicine nécessite une manipulation délicate et peu reproductible, qui va à l'encontre de la sécurité et de la qualité des soins.

Il est ainsi apparu nécessaire d'offrir un espaceur articulaire prêt à l'emploi ne requérant pas de manipulation complexe en salle d'opération, et permettant d'apporter au moins deux antibiotiques en traitement local, ceci sans augmenter sensiblement les coûts de fabrication. Or, on sait que le mélange de composés en poudre est délicat et pose des problèmes d'homogénéité.

L'espaceur objet de la présente invention apporte une solution à ces exigences en proposant un dispositif composé de deux pièces complémentaires susceptibles d'être assemblées d'un geste pour former un implant complet comprenant deux antibiotiques différents qui permettront un traitement local efficace des infections de la zone articulaire.

Outre que la présente invention fournit un espaceur prêt à l'emploi, apportant au moins deux substances actives différentes, elle présente d'autres avantages découlant de la modularité du dispositif. Elle offre en particulier la possibilité de choisir les deux antibiotiques à associer à partir d'une gamme de pièces complémentaires. Elle permet aussi de disposer d'une gamme d'espaceurs de tailles différentes, sans avoir à conserver un stock important.

Un autre avantage de l'invention est de disposer d'un espaceur pouvant être fabriqué en série, sans allonger ou alourdir le processus technologique, de manière à rester dans des prix de revient raisonnables. En particulier, il est avantageux d'éviter les difficultés technologiques dans le procédé de fabrication liés à l'homogénéisation des mélanges de poudres, forme sous laquelle on apporte généralement les substances actives dans le ciment. Ceci est résolu par la présente invention par le fait que l'on introduit un seul des composés actifs en poudre dans les ingrédients du ciment destiné à la fabrication d'un des éléments de l'espaceur. On réduit ainsi le problème de la répartition homogène des substances actives ainsi que celui de l'uniformité des propriétés mécaniques des ciments obtenus. Un autre avantage de l'invention est de pouvoir contrôler facilement les caractéristiques mécaniques et les propriétés du ciment chargé en substance active en évitant d'avoir à modéliser le comportement des mélanges à composants multiples.

Ainsi, la présente invention a pour objet un dispositif espaceur en ciment polymère pour le remplacement d'une prothèse articulaire permanente lors d'une reprise en deux temps, comprenant une tige apte à être fixée à l'os support et une tête apte à se placer dans la zone articulaire, ledit dispositif espaceur étant constitué de deux éléments complémentaires comprenant des moyens d'assemblage rigide entre eux, le premier élément étant chargé d'une première substance active, et le deuxième élément étant chargé d'une deuxième substance active.

Le dispositif selon l'invention peut adopter n'importe quelle forme utilisable pour réaliser un espaceur provisoire, qu'elle soit connue de l'homme de l'art à ce jour ou non, celle-ci étant imposée en grande partie par l'anatomie de l'articulation à traiter. On désignera dans la présente demande par "articulation" l'ensemble constituée par les régions voisines de deux os coopérant pour assurer une liaison flexible du squelette. Il peut s'agit d'une articulation de type charnière telle que le genoux où les têtes de deux os glissent l'une sur l'autre lors de la flexion, ou bien d'une articulation de type enarthose, dite aussi "presque sphère et cupule", comme la hanche. Dans ce cas, la tête du fémur, ou presque sphère, est maintenue dans la cavité cotyloïdienne de l'os du bassin et glisse à l'intérieur de la cupule. L'espaceur selon l'invention est destiné à remplacer une extrémité osseuse, que celle-ci coopère avec une autre extrémité osseuse ou avec une cupule. La réparation osseuse de la hanche étant de loin la plus répandue, elle sera spécifiquement décrite ici, bien que la présente invention soit explicitement destinée à la réparation de toute articulation susceptible de recevoir un implant provisoire.

Par analogie avec le vocabulaire anatomique, on appelle "tête" de l'espaceur l'extrémité arrondie destinée à remplacer la tête osseuse. La tige est la partie destinée à être insérée dans l'os support, et pouvant en outre se substituer partiellement à lui pour apporter la longueur nécessaire. La tête et la tige forment un ensemble, à savoir l'espaceur, leur jonction étant assurée par un simple changement de section de la pièce, avec ou sans changement d'orientation.

Les moyens d'assemblage de deux éléments complémentaires de l'espaceur selon l'invention peuvent en principe être placés à un niveau quelconque de l'espaceur, par exemple au milieu de la tige. Dans un mode préféré de réalisation de l'espaceur selon l'invention, les moyens d'assemblage de deux éléments complémentaires sont placés à la jonction de la tige et de la tête de l'espaceur. Cette caractéristique procure différents avantages qui apparaîtront mieux par la suite, tels que choix des éléments en fonction de leur dimension ou de leur chargement, facilité de fabrication et de montage, ....

De manière avantageuse, les moyens d'assemblage sont choisis parmi les moyens d'assemblage aptes à être unis manuellement, avec ou sans l'aide d'un outil. Il est ainsi aisé pour le chirurgien de réaliser d'un seul geste le montage de l'espaceur à partir de deux éléments, instantanément. Les moyens d'assemblage peuvent être par exemple constitués par un cône morse, le cône mâle étant de préférence porté par la tige et le cône femelle par la tête. Lorsque les moyens d'assemblage sont unis par coincement d'un élément dans l'autre, la force nécessaire peut être fournie par un outil d'appoint tel qu'un impacteur. L'assemblage est fait de préférence par simple pression manuelle.

Dans ce dernier mode de réalisation, selon une autre caractéristique intéressante de l'espaceur selon l'invention, la tête présente une symétrie de révolution par rapport à l'axe de la jonction. En effet, dans ce cas sa position relative des pièces lors de l'assemblage peut être quelconque. L'orientation relative des deux éléments est déterminée lors du moulage des pièces par la forme de la jonction. Ainsi, le praticien peut procéder au montage de l'espaceur très rapidement et en toute tranquillité quant au résultat de l'assemblage.

Comme déjà indiqué, la forme et les dimensions des espaceurs sont en grande partie imposées par l'anatomie de l'articulation à traiter. Comme la stature des patients est variable, il est utile que l'espaceur selon l'invention puisse adopter plusieurs formats, et en particulier que les dimensions de la tête et de la tige puissent varier indépendamment l'une de l'autre pour être associées selon toutes les combinaisons souhaitées. Ainsi, en particulier lorsque l'espaceur est destiné à être placé dans la hanche, la tige peut présenter une forme sensiblement cylindrique ou conique de longueur comprise entre 80 mm et 200 mm. En outre, la tête peut s'inscrire dans une sphère de diamètre compris entre 40 mm et 70 mm.

Selon un mode de réalisation préféré du dispositif espaceur selon l'invention, la tige comprend en son âme une armature métallique. Cette armature permet de reprendre les efforts appliqués sur l'espaceur et de rigidifier l'ensemble.

La structure de l'espaceur telle qu'elle vient d'être décrite présente en soi de nombreux avantages, notamment en ce qui concerne la possibilité de combiner des éléments de tailles différentes, adaptés à l'anatomie spécifique de chaque patient. On peut donc fort bien utiliser de tels espaceurs en deux éléments assemblés, qu'ils soient chargés d'une substance active ou neutres. Toutefois, la structure en deux éléments est essentiellement destinée à être chargée de deux actifs que l'on souhaite délivrer en traitement local. On note au passage que si le besoin d'un traitement par trois actifs (ou même davantage) se manifestait, il serait tout à fait possible de réaliser un espaceur polymère formé de trois éléments chargés de trois substances actives différentes.

La structure décrite ici est particulièrement indiquée pour résoudre le problème des infections résistantes par traitement local de la zone articulaire par deux antibiotiques distincts. C'est pourquoi, selon un mode particulièrement préféré de réalisation de l'espaceur selon l'invention, les première et deuxième substances actives sont des antibiotiques. Ils peuvent notamment être choisis parmi la gentamicine, la trobamycine, la vancomycine, l'érythromycine, la fucidine, la tétracycline, ou d'autres antibiotiques convenables.

De manière avantageuse, la première substance active est choisie parmi les antibiotiques à spectre large et la deuxième substance active est choisie parmi les antibiotiques à activité ciblée. Ce choix, justifié par l'expérience des cliniciens, permet d'apporter la meilleure chance de succès du traitement.

Selon un mode de réalisation particulier, le ciment polymère composant la tige comprend avantageusement de 1,5 % à 4,5 % de gentamicine en poids rapporté au poids de ciment, et de préférence environ 2,3 %. Des prémélanges pour la préparation d'un ciment incorporant la gentamicine sont disponibles dans le commerce, par exemple le ciment Gentafix™ commercialisé par le demandeur.

Par ailleurs, le ciment polymère composant la tête peut avantageusement comprendre de la vancomycine à raison de 0,5 % à 3 % en poids rapporté au poids de ciment, de préférence à hauteur d'environ 1,25%.

Les deux éléments sont préformés et sont fabriqués à base d'un matériau polymère entièrement biocompatible et poreux. Les ciments acryliques à base de polyméthyl-méthacrylate de méthyle, bien connus de l'homme de l'art, puisqu'il sont utilisés communément pour fabriquer les espaceurs monoblocs du commerce ainsi que les ciments de scellement en ostéoplastie, sont tout à fait adaptés à la fabrication de l'espaceur selon l'invention.

Un tel ciment est préparé à partir du mélange d'un prépolymère, généralement du PMMA (polyméthylméthacrylate de méthyle) et d'un monomère, généralement du MMA (méthylméthacrylate de méthyle) pouvant être accompagné d'un co-monomère (butryl), réagissant en présence d'un activateur de polymérisation. Ils contiennent également des additifs technologiques tels qu'un activateur chimique, un initiateur, un stabilisant. Afin de visualiser l'espaceur durant et après l'opération par des moyens radiologiques, une substance radio-opaque peut aussi être ajoutée.

Différents ciments de ce type sont disponibles dans le commerce. Ils sont communément présentés sous forme de deux composants séparés: une poudre comprenant principalement des billes de prépolymère, et un liquide contenant principalement le monomère, comme par exemple le ciment Cemfix™ commercialisé par le demandeur. Étant employés pour la fabrication d'espaceurs et pour d'autres applications en ostéochirurgie, ils répondent à des critères de résistance à la traction et à la compression, de neutralité chimique et de biocompatibilité. Les additifs présents dans le ciment final n'ont pas d'effet biologique indésirable. Ces ciments ont prouvé leurs qualités et sont homologués pour un usage médical.

L'antibiotique choisi est incorporé sous forme de poudre sèche au composant polymère en poudre et soigneusement mêlé avant mise en présence avec le composant liquide. Durant le processus de polymérisation, il est piégé dans le réseau moléculaire. Lorsque l'espaceur sera assemblé et installé dans l'articulation, chacun des antibiotiques sera libéré progressivement au contact des fluides corporels. Le moulage des pièces est réalisé selon les techniques connues de l'homme de l'art, par exemple dans des moules en silicone.

L'antibiotique présent dans le ciment de chaque élément de l'espaceur selon l'invention, va diffuser vers les tissus proches pour empêcher l'apparition ou l'extension de l'infection dans là zone d'implantation. On note que cette technique permet d'éviter les interférences dans le libération par le PMMA des deux antibiotiques et d'ajuster la vitesse d'élution pour chaque antibiotique indépendamment. De manière avantageuse, le dispositif espaceur selon l'invention contient au total, de 0,8 g à 1,6 g de gentamicine et de 0,5 g à 1,5 g, de vancomycine.

Grâce à la présente invention, un établissement hospitalier pourra disposer d'une gamme composée de têtes et de tiges comprenant différents antibiotiques ou autres substances actives, de tailles différentes et pouvant être combinés selon les besoins. Cette modularité permet à l'équipe soignante de choisir, suivant l'infection, l'antibiotique spécifique, dans la taille requise, sans qu'il soit nécessaire de disposer d'un stock important. Surtout, ceci permet de mettre en oeuvre un traitement plus efficace pour le patient et de donner plus de liberté d'action aux soignants, tout en leur fournissant du matériel de qualité, très homogène, dépourvu de faiblesses mécaniques et assurant une diffusion régulière des actifs.

Les exemples qui suivent illustrent l'invention afin d'en faire mieux apparaître les caractéristiques et les avantages, sans toutefois en réduire en quelque manière la portée.
La figure 1 représente un espaceur de hanche selon l'invention, avant assemblage.
La figure 2 représente un espaceur de hanche selon l'invention, après assemblage.

Les abréviations suivantes sont utilisées:

| | |
|---|---|
| PMMA: | polyméthacrylate de méthyle |
| MMA: | méthacrylate de méthyle |
| BPO : | péroxyde de benzoyle |
| BaSO₄: | sulfaté de barium |
| DMTP: | diméthyl-par-a-toluidine |
| HQ: | hydroquinone |

### EXEMPLE 1: Structure d'un espaceur de hanche

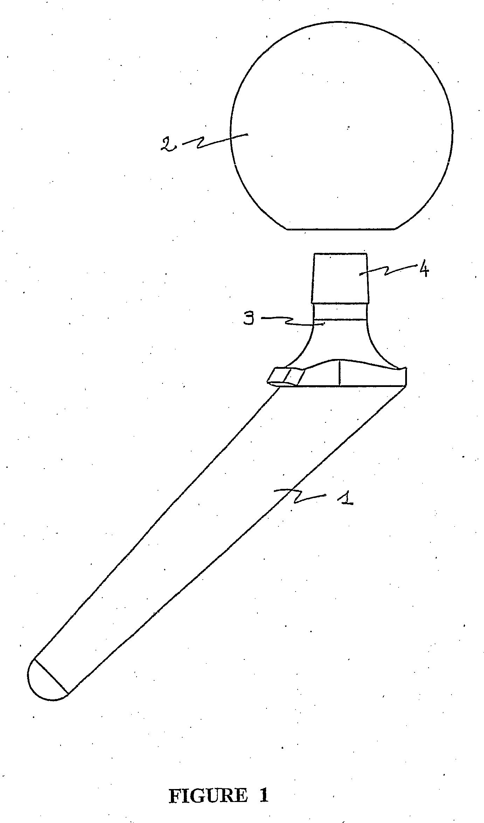

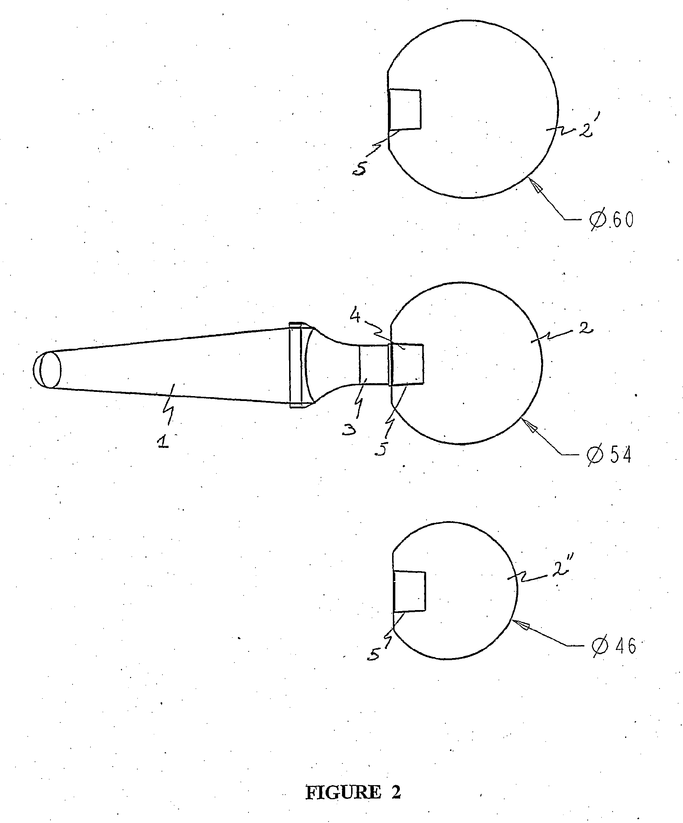

Le dispositif espaceur en ciment polymère pour le remplacement d'une prothèse de hanche tel que représenté sur les Figures 1 et 2, comprend :
- la tige 1 apte à être fixée à l'os support, ici le fémur, constituant un premier élément chargé d'une première substance active, et
- la tête 2 apte à se placer dans la zone articulaire, constituant un deuxième élément chargé d'une deuxième substance active.

Ces deux éléments sont complémentaires et comprennent les moyens d'assemblage rigide entre eux placés à la jonction 3 de la tige 1 et de la tête 2 de l'espaceur. Les moyens d'assemblage sont constitués par un cône morse, le cône mâle 4 étant porté par la tige 1 et le cône femelle 4 par la tête 2, qui permet l'assemblage de l'espaceur par simple pression manuelle, ou à l'aide d'un impacteur.

La tige 1 présente une forme sensiblement conique et se termine à son extrémité proximale par la jonction 3 comportant le cône mâle 4. Elle a une longueur de 120 mm et un diamètre de 20 mm. Elle comprend en son âme une armature métallique (non représentée) d'une section variable de 6 mm à 10 mm, La tête 2 présente une symétrie de révolution par rapport à l'axe du cône morse. Elle s'inscrit dans une sphère d'un diamètre de 54 mm (2), ou alternativement de 60 mm (2') ou de 46 mm (2").

### EXEMPLE 2: Composition d'un ciment C1

La composition C1 correspond à un ciment polymère contenant de la gentamicine sous forme de sulfate, en tant qu'antibiotique à spectre large.

| PHASE POUDRE (% en poids) | | PHASE LIQUIDE (% en poids) | |
|---|---|---|---|
| PMMA: | 84,3 % | MIMA: | 84,4 % |
| BPO: | 2,3 % | Butryl: | 13,2 % |
| BaSO₄: | 9,6 % | DMPT: | 2,4 % |
| Sulfate de gentamicine : | 3,8 % | HQ: | 20 ppm |

Le ciment est préparé par mélange dans un réacteur, de 40 kg de phase poudre avec 25 kg de phase liquide. Quand le mélange est homogène et de consistance crémeuse, il est coulé dans les moules de fabrication. Après, séchage et démoulage, les pièces sont ébavurées. Elles peuvent alors être stérilisées et conditionnées selon une méthode connue.

On obtient des pièces contenant la gentamicine à hauteur de 2,34 % en poids.

### EXEMPLE 3 : Composition d'un ciment C2

La composition C2 correspond à un ciment polymère contenant de la vancomycine, en tant qu'antibiotique à action ciblée. Elle agit sur les germes tels que les espèces aérobies à Gram positif (les Bacilles, les Entérocoques, *Listera Rhodocaccus equi, Staphylococcus aureus,* les Staphylocoques non aureus, les Streptocoques notamment *Streptococcus pneumonia*), et sur les espèce anaérobies (Clostridium, Eubactérium, Peptostreptocoques, *Propionibacterium acnés*).

| PHASE POUDRE (% en poids) | | PHASE LIQUIDE (% en poids) | |
|---|---|---|---|
| PMMA : | 87,6 % | MMA: | 84,4 % |
| BPO: | 2,4 % | Butryl: | 13,2 % |
| BaSO₄: | 10,0 % | DMPT: | 2,4 % |
| Vancomycine: | 4,8 % | HQ: | 20ppm |

Le ciment C2 est préparé selon le même protocole que celui décrit ci-dessus à l'exemple 2, à partir de 42 kg de phase poudre et de 25 kg de phase liquide.

On obtient des pièces contenant la vancomycine à hauteur de 1,25 % en poids.

### EXEMPLE 4 : Composition d'un ciment C3

La composition C3 correspond à un ciment polymère contenant de la trobamycine, en tant qu'antibiotique à action ciblée. Elle agit sur les germes tels que les espèces aérobies à Gram positif (Corynebacterium, *Listeria monocytogenes*, *Staphylococcus aureus* méticilline-sensible, Staphylocoques coagulase-négative méticilline-sensible), et sur les espèce aérobies à Gram négatif (Acinobacter notamment *A. baumanii, Branhamella catarrhalis,* Campylobacter, *Citrobacter freundii, Citrobacter koseri, Enterobacter aerogenes, Enterobacter cloacae, Escherichia coli, Haemophilus influenzae,* Klebsiella, *Morganella morganii, Proteus mirabilis, Proteus vulgaris, Pseudomonas aeruginosa,* Salmonella, Serratia, Shigella, Yersinia)

| PHASE POUDRE (% en poids) | | PHASE LIQUIDE (% en poids) | |
|---|---|---|---|
| PMMA: | 87,6 % | MMA: | 84,4 % |
| BPO: | 2,4 % | Butryl : | 13,2 % |
| BaSO₄: | 10,0 % | DMPT : | 2,4 % |
| Trobamycine: | 4,8 % | HQ: | 20ppm |

Le ciment C3 est préparé comme décrit ci-dessus à l'exemple 2, à partir de 42 kg de phase poudre et de 25 kg de phase liquide.

On obtient des pièces contenant la trobamycine à hauteur de 1,25 % en poids.

### EXEMPLE 5: Espaceur de hanche E1

L'espaceur de hanche E1 est fabriqué à l'aide d'une tige obtenue à l'aide du ciment C1 contenant de la gentamicine, et d'une tête obtenue à l'aide du ciment C2 contenant de la vancomycine. Des tests de diffusion ont été réalisés.

La cinétique de diffusion de la gentamicine est bien connue de l'homme de l'art puisque cet antibiotique est déjà administré à l'aide des espaceurs monoblocs usuels. Elle se manifeste par un apport important durant les premières heures puis la libération ralentit dans les deux semaines suivant l'implantation, et reste quasiment constante par la suite durant plusieurs semaines. Cet effet "flash" est bénéfique pour amorcer le traitement efficacement.

Les tests effectués sur la diffusion de la vancomycine ont montré que 3% de la vancomycine initiale ont été libérés après une semaine d'implantation de l'espaceur, dont 15 mg le premier jour, 3 mg le deuxième jour et 1,3 mg le troisième jour. Après 6 mois, c'est 8 % de la vancomycine qui a été diffusée.

De manière générale les tests montrent une bonne diffusion de la vancomycine dans l'espace articulaire. En outre si le mélange des poudres est soigneusement réalisé, on n'observe quasiment pas d'altération du ciment d'un point de vue mécanique.

## Revendications

1. Dispositif espaceur en ciment polymère pour le remplacement d'une prothèse articulaire permanente lors d'une reprise en deux temps ***caractérisé en ce qu***'il est constitué de deux éléments complémentaires (1, 2) comprenant des moyens d'assemblage rigide entre eux placés à un niveau quelconque de l'espaceur, et en ce que le premier élément est chargé d'une première substance active, et le deuxième élément est chargé d'une deuxième substance active, différente de ladite première substance active.

2. Dispositif espaceur selon la revendication 1 ***caractérisé en ce que*** les deux éléments complémentaires consistent respectivement en une tige (1) apte à être fixée à un os support et une tête (2) apte à se placer dans la zone articulaire, et les moyens d'assemblage sont placés à la jonction (3) de la tige (1) et de la tête (2) de l'espaceur.

3. Dispositif espaceur selon la revendication 1 ou 2, ***caractérisé en ce que*** les moyens, d'assemblage sont choisis parmi les moyens d'assemblage aptes à être unis manuellement, avec ou sans l'aide d'un outil.

4. Dispositif espaceur selon l'une des revendications 2 à 3 ***caractérisé en ce que*** la tête (2) présente une symétrie de révolution par rapport à l'axe de la jonction (3).

5. Dispositif espaceur selon l'une des revendications précédentes ***caractérisé en ce que*** la tige (1) comprend en son âme une armature métallique.

6. Dispositif espaceur selon l'une quelconque des revendications précédentes ***caractérisé en ce que*** les première et deuxième substances actives sont des antibiotiques.

7. Dispositif espaceur selon la revendication 6 ***caractérisé en ce que*** les première et deuxième substances actives sont choisies parmi les antibiotiques suivants : la gentamicine, la trobamycine, l'érythomycine, la vancomycine, la fucidine, la tétracycline.

8. Dispositif espaceur selon la revendication 7 ***caractérisé en ce que*** la première substance active est choisie parmi les antibiotiques à spectre large et la deuxième substance active est choisie parmi les antibiotiques à activité ciblée.

9. Dispositif espaceur selon la revendication précédente ***caractérisé en ce que*** le ciment polymère composant la tige (1) comprend de 1,5 % à 4,5 % de gentamicine en poids rapporté au poids de ciment, et de préférence environ 2,3 %.

10. Dispositif espaceur selon la revendication 8 ***caractérisé en ce que*** le ciment polymère composant la tête (2) comprend de 0,5 % à 3 % de vancomycine en poids rapporté au poids de ciment, et de préférence environ 1,25 %.

11. Dispositif espaceur selon l'une des revendications 1 à 10 ***caractérisé en ce qu***'il contient au total de 0,8 g à 1,6 g de gentamicine et de 0,5 g à 1,5 g de vancomycine.

## Patentansprüche

1. Abstandshaltevorrichtung aus Polymerzement für das Ersetzen einer Dauergelenkprothese anlässlich einer zweistufigen Entnahme,
**dadurch gekennzeichnet,**
**dass** sie aus zwei komplementären Elementen (1, 2) gebildet ist, die zwischen einander starre Mittel zum Zusammensetzen beinhalten, die auf einem beliebigen Niveau des Abstandshalters angeordnet sind, und
**dass** das erste Element eine erste aktive Substanz trägt und das zweite Element eine zweite aktive Substanz, die sich von der ersten aktiven Substanz unterscheidet, trägt.

2. Abstandshaltevorrichtung nach Anspruch 1,
**dadurch gekennzeichnet, dass**
die beiden komplementären Elemente aus einem Stift (1), der dazu ausgebildet ist, an einem Knochenträger fixiert zu werden, beziehungsweise einem Kopf (2), der dazu ausgebildet ist, sich in der Gelenkzone anzuordnen, bestehen,
und die Mittel zum Zusammensetzen an der Verbindung (3) des Stifts (1) und des Kopfs (2) des Abstandshalters angeordnet sind.

3. Abstandshaltevorrichtung nach Anspruch 1 oder 2,
**dadurch gekennzeichnet, dass**
die Mittel zum Zusammensetzen ausgewählt sind aus der Mitteln zum Zusammensetzen, die dazu ausgebildet sind, manuell mit oder ohne die Hilfe eines Werkzeugs vereinigt zu werden.

4. Abstandshaltevorrichtung nach Anspruch 2 oder 3,
dass der Kopf (2) eine Drehsymmetrie bezüglicher der Verbindungsachse (3) aufweist.

5. Abstandshaltevorrichtung nach einem der vorhergehenden Ansprüche,
**dadurch gekennzeichnet,**
**dass** der Stift (1) in seinem Inneren eine metallische Armatur aufweist.

6. Abstandshaltevorrichtung nach einem der vorhergehenden Ansprüche,
**dadurch gekennzeichnet,**
**dass** die erste und die zweite aktive Substanz Antibiotika sind.

7. Abstandshaltevorrichtung nach Anspruch 6,
**dadurch gekennzeichnet,**
**dass** die erste und die zweite aktive Substanz ausgewählt sind aus den folgenden Antibiotika: Gentamicin, Tobramicin, Erythromycin, Vancomycin, Fusidinsäure, Tetracyclin.

8. Abstandshaltevorrichtung nach Anspruch 7,
**dadurch gekennzeichnet,**
**dass** die erste aktive Substanz ausgewählt ist aus den Breitbandantibiotika und die zweite aktive Substanz ausgewählt ist aus den Antibiotika mit zielgerichteter Aktivität.

9. Abstandshaltevorrichtung nach dem vorhergehenden Anspruch,
**dadurch gekennzeichnet,**
**dass** der Polymerzement, aus dem der Stift (1) besteht, zwischen 1,5 und 4,5 Gewichtsprozent Gentamicin bezogen auf das Gewicht des Zements, und bevorzugt etwa 2,3 % aufweist.

10. Abstandshaltevorrichtung nach dem Anspruch 8,
**dadurch gekennzeichnet,**
**dass** der Polymerzement, aus dem der Kopf (2) zusammengesetzt ist, zwischen 0,5 und 3 Gewichtsprozent Vancomycin bezogen auf das Gewicht des Zements enthält und vorzugsweise 1,25 %.

11. Abstandshaltevorrichtung nach einem der Ansprüche 1 bis 10,
**dadurch gekennzeichnet,**
**dass** sie insgesamt 0,8 g bis 1,6 g Gentamicin und 0,5 g bis 1,5 g Vancomycin enthalten.

## Claims

1. Spacer device of polymer cement, for the replacement of a permanent joint prosthesis in the case of a two-stage replacement, ***characterized in that*** it is composed of two complementary members (1, 2) incorporating rigid means of assembly to one another, placed at any level of the spacer, and **in that** the first member is loaded with a first active substance and the second member is loaded with a second active substance different from said first active substance.

2. Spacer device according to claim 1, ***characterized in that*** the two complementary members respectively consist of a shank (1) suitable for attachment to a supporting bone and a head (2) suitable for placement in a joint region, and the means of assembly are located at the junction (3) between the shank (1) and the head (2) of the spacer.

3. Spacer device according to claim 1 or 2, ***characterized in that*** the means of assembly are selected from means of assembly suitable for being joined manually, with or without the assistance of a tool.

4. Spacer device according to any one of claims 2 to 3, ***characterized in that*** the head (2) is rotationally symmetrical relative to the axis of the junction (3).

5. Spacer device according to any one of the preceding claims, ***characterized in that*** the shank (1) comprises a metal reinforcement in its core.

6. Spacer device according to any one of the preceding claims, ***characterized in that*** the first and second active substances are antibiotics.

7. Spacer device according to claim 6, ***characterized in that*** the first and second active substances are selected from the following antibiotics: gentamicin, tobramycin, erythromycin, vancomycin, fucidin, and tetracycline.

8. Spacer device according to claim 7, ***characterized in that*** the first active substance is selected from broad spectrum antibiotics and the second active substance is selected from antibiotics having targeted activity.

9. Spacer device according to the preceding claim, ***characterized in that*** the polymer cement composing the shank (1) comprises from 1.5% to 4.5% gentamicin by weight in relation to the weight of the cement, and preferably approximately 2.3%.

10. Spacer device according to claim 8, ***characterized in that*** the polymer cement composing the head (2) comprises from 0.5% to 3% vancomycin by weight in relation to the weight of the cement, and preferably about 1.25%.

11. Spacer device according to any one of claims 1 to 10, ***characterized in that*** it contains a total of 0.8 g to 1.6 g gentamicin and 0.5 g to 1.5 g vancomycin.
